# EUROPEAN PATENT SPECIFICATION

(11) **EP 2 598 877 B1**
(45) Date of publication and mention of the grant of the patent: **19.04.2017**
(21) Application number: 11733660.2
(22) Date of filing: 11.07.2011
(51) Int. Cl.: C12N 5/0793, G01N 33/50, G01N 33/68, G01N 33/84, A23K 20/142, A23K 20/26, A23K 50/40, A23K 50/48

(54) **METHODS AND COMPOSITIONS FOR STIMULATING A FELIDAE T1R RECEPTOR**
VERFAHREN UND ZUSAMMENSETZUNGEN ZUR STIMULATION EINES T1R-REZEPTORS DER FELIDAE
PROCÉDÉS ET COMPOSITIONS POUR LA STIMULATION D'UN RÉCEPTEUR T1R DE FÉLIDÉS

(30) Priority: 27.07.2010 US 367930 P
(43) Date of publication of application: 05.06.2013
(73) Proprietor: Spécialités Pet Food, 56250 Elven (FR); Monell Chemical Senses Center, Philadelphia, PA 19104-3308 (US)
(72) Inventor: BRAND, Joseph G., Wayne, Pennsylvania 19087 (US); BRYANT, Bruce, Elkins Park, Pennsylvania 19027 (US)
(74) Representative: Regimbeau
(86) International application number: PCT/EP2011/061786
(87) International publication number: WO 2012/013480

(56) References cited:
- WO-A1-2007/118876
- WO-A2-2005/005480
- US-A1- 2008 244 761

### SEQUENCE LISTING

<110> SPECIALITES PET FOOD
   MONELL CHEMICAL SENSES CENTER
   BRAND, Joseph
   BRYANT, Bruce
<120> METHODS AND COMPOSITIONS FOR STIMULATING A FELIDAE T1R RECEPTOR
<130> 358581D29885
<150> US 61/367930
   <151> 2010-07-27
<160> 4
<170> PatentIn version 3.5
<210> 1
   <211> 841
   <212> PRT
   <213> Felis catus
<400> 1
   Met Ser Leu Pro Ala Ala His Leu Val Gly Leu Gln Leu Ser Leu Ser
   1               5                   10                  15
   Cys Cys Trp Ala Leu Ser Cys His Ser Thr Glu Thr Ser Ala Asp Phe
               20                  25                  30
   Ser Leu Pro Gly Asp Tyr Leu Leu Ala Gly Leu Phe Pro Leu His Ser
           35                  40                  45
   Asp Cys Pro Gly Val Arg His Arg Pro Thr Val Thr Leu Cys Asp Arg
   50 55 60
   Pro Asp Ser Phe Asn Gly His Gly Tyr His Leu Phe Gln Ala Met Arg
   65 70 75 80
   Phe Gly Ile Glu Glu Ile Asn Asn Ser Thr Ala Leu Leu Pro Asn Val
   85 90 95
   Thr Leu Gly Tyr Gln Leu Tyr Asp Val Cys Ser Glu Ser Ala Asn Val
   100 105 110
   Tyr Ala Thr Leu Asn Val Leu Ser Leu Leu Gly Thr His His Val Glu
   115 120 125
   Ile Arg Ala Asp Pro Ser His Tyr Ser Pro Ala Ala Leu Ala Val Ile
   130 135 140
   Gly Pro Asp Thr Thr Asn His Ala Ala Thr Thr Ala Ala Leu Leu Ser
   145 150 155 160
   Pro Phe Leu Val Pro Leu Ile Ser Tyr Glu Ala Ser Ser Val Thr Leu
   165 170 175
   Gly Val Lys Arg His Tyr Pro Ser Phe Leu Arg Thr Ile Pro Ser Asp
   180 185 190
   Lys His Gln Val Glu Ala Met Val Leu Leu Leu Gln Ser Phe Gly Trp
   195 200 205
   Val Trp Ile Ser Val Val Gly Ser Asp Gly Asp Tyr Gly Gln Leu Gly
   210 215 220
   Val Gln Ala Leu Glu Glu Gln Ala Thr Gln Gln Gly Ile Cys Val Ala
   225 230 235 240
   Phe Lys Asp Ile Ile Pro Phe Ser Ala Arg Pro Gly Asp Glu Arg Met
   245 250 255
   Gln Ser Ile Met His His Leu Ala Arg Ala Arg Thr Thr Val Val Val
   260 265 270
   Val Phe Ser Ser Arg Gln Leu Ala Arg Val Phe Phe Glu Ser Val Val
   275 280 285
   Leu Ala Asn Leu Thr Ala Lys Val Trp Ile Ala Ser Glu Asp Trp Ala
   290 295 300
   Ile Ser Arg His Ile Ser Asn Val Pro Gly Ile Gln Gly Ile Gly Thr
   305                 310                 315                 320
   Val Leu Gly Val Ala Ile Gln Gln Arg Leu Val Pro Gly Leu Lys Glu
                   325                 330                 335
   Phe Glu Glu Ala Tyr Val Gln Ala Asp Lys Gly Ala Pro Gly Pro Cys
               340                 345                 350
   Ser Arg Thr Ser Glu Cys Ser Ser Asn Gln Leu Cys Arg Glu Cys Arg
           355                 360                 365
   Ala Phe Thr Ala Glu Gln Met Pro Thr Leu Gly Ala Phe Ser Met Ser
       370                 375                 380
   Ser Ala Tyr Asn Ala Tyr Arg Ala Val Tyr Ala Val Ala His Gly Leu
   385                 390                 395                 400
   His Gln Leu Leu Gly Cys Ala Ser Gly Ala Cys Ser Arg Asp Arg Val
                   405                 410                 415
   Tyr Pro Trp Gln Leu Leu Glu Gln Ile Arg Lys Val Asn Phe Leu Leu
               420                 425                 430
   His Lys Asp Thr Val Arg Phe Asn Asp Asn Gly Asp Pro Leu Ser Gly
   435 440 445
   Tyr Asp Ile Ile Ala Trp Asp Trp Ser Gly Pro Lys Trp Asn Phe Arg
   450 455 460
   Val Ile Gly Ser Ser Met Trp Pro Pro Val Gln Leu Asp Ile Asn Lys
   465 470 475 480
   Thr Lys Ile Arg Trp His Gly Lys Asp Asn Gln Val Pro Lys Ser Val
   485 490 495
   Cys Ser Ser Asp Cys Leu Glu Gly His Gln Arg Val Ile Ser Gly Phe
   500 505 510
   Tyr His Cys Cys Phe Glu Cys Val Pro Cys Glu Ala Gly Ser Phe Leu
   515 520 525
   Asn Lys Ser Asp Leu His Ser Cys Gln Pro Cys Gly Lys Glu Lys Trp
   530 535 540
   Ala Pro Ala Gly Ser Glu Thr Cys Phe Pro Arg Thr Val Val Phe Leu
   545 550 555 560
   Thr Trp His Glu Thr Ile Ser Trp Val Leu Leu Ala Ala Asn Thr Leu
   565 570 575
   Leu Leu Leu Leu Val Thr Gly Thr Ala Gly Leu Phe Ala Trp His Leu
   580 585 590
   Asp Thr Pro Val Val Lys Ser Ala Gly Gly Arg Leu Cys Phe Phe Met
   595 600 605
   Leu Gly Ser Leu Ala Gly Gly Ser Cys Gly Leu Tyr Gly Phe Phe Gly
   610 615 620
   Glu Pro Thr Leu Pro Thr Cys Leu Leu Arg Gln Ser Leu Leu Ala Leu
   625 630 635 640
   Gly Phe Ala Ile Phe Leu Ser Cys Leu Thr Ile Arg Ser Phe Gln Leu
   645 650 655
   Val Phe Ile Phe Lys Phe Ser Ala Lys Val Pro Thr Phe Tyr Arg Ala
   660 665 670
   Trp Val Gln Asn His Gly Pro Gly Leu Phe Val Val Ile Ser Ser Met
   675 680 685
   Ala Gln Leu Leu Ile Cys Leu Thr Trp Leu Ala Val Trp Thr Pro Leu
   690 695 700
   Pro Thr Arg Glu Tyr Gln Arg Phe Pro Gln Leu Val Val Leu Asp Cys
   705 710 715 720
   Thr Glu Ala Asn Ser Pro Gly Phe Met Leu Ala Phe Ala Tyr Asn Gly
   725 730 735
   Leu Leu Ser Val Ser Ala Phe Ala Cys Ser Tyr Leu Gly Lys Asp Leu
   740 745 750
   Pro Glu Asn Tyr Asn Glu Ala Lys Cys Val Thr Phe Ser Leu Leu Leu
   755 760 765
   Asn Phe Val Ser Trp Ile Ala Phe Phe Thr Thr Ala Ser Val Tyr Gln
   770 775 780
   Gly Lys Tyr Leu Pro Ala Val Asn Val Leu Ala Ala Leu Ser Ser Leu
   785 790 795 800
   Ser Gly Gly Phe Ser Gly Tyr Phe Leu Pro Lys Cys Tyr Val Ile Leu
   805 810 815
   Cys Arg Pro Lys Phe Asn Ser Thr Gln His Phe Gln Ala Ser Ile Gln
   820 825 830
   Glu Tyr Thr Arg Arg Cys Gly Ser Thr
   835 840
<210> 2
   <211> 865
   <212> PRT
   <213> Felis catus
<400> 2
   Met Pro Gly Leu Ala Leu Leu Gly Leu Thr Ala Leu Leu Gly Leu Thr
   1 5 10 15
   Ala Leu Leu Asp His Gly Glu Gly Ala Thr Ser Cys Leu Ser Gln Gln
   20 25 30
   Leu Arg Met Gln Gly Asp Tyr Val Leu Gly Gly Leu Phe Pro Leu Gly
   35 40 45
   Ser Ala Glu Gly Thr Gly Leu Gly Asp Gly Leu Gln Pro Asn Ala Thr
   50 55 60
   Val Cys Thr Arg Phe Ser Ser Leu Gly Leu Leu Trp Ala Leu Ala Val
   65 70 75 80
   Lys Met Ala Val Glu Glu Ile Asn Asn Gly Ser Ala Leu Leu Pro Gly
                   85                  90                  95
   Leu His Leu Gly Tyr Asp Leu Phe Asp Thr Cys Ser Glu Pro Met Val
               100                 105                 110
   Ala Met Lys Pro Ser Leu Val Phe Met Ala Lys Ala Gly Ser Cys Ser
           115                 120                 125
   Ile Ala Ala Tyr Cys Asn Tyr Thr Gln Tyr Gln Pro Arg Val Leu Ala
       130                 135                 140
   Val Ile Gly Pro His Ser Ser Glu Leu Ala Leu Val Thr Gly Lys Phe
   145                 150                 155                 160
   Phe Ser Phe Phe Leu Val Pro Gln Val Ser Tyr Gly Ala Ser Thr Asp
                   165                 170                 175
   Arg Leu Ser Asn Arg Glu Ile Phe Pro Ser Phe Phe Arg Thr Val Pro
               180                 185                 190
   Ser Asp Gln Val Gln Val Ala Ala Met Val Glu Leu Leu Glu Glu Leu
           195                 200                 205
   Gly Trp Asn Trp Val Ala Ala Val Gly Ser Asp Asp Glu Tyr Gly Arg
   210 215 220
   Gln Gly Leu Ser Leu Phe Ser Gly Leu Ala Ser Ala Arg Gly Ile Cys
   225 230 235 240
   Ile Ala His Glu Gly Leu Val Pro Leu Pro Pro Gly Ser Leu Arg Leu
   245 250 255
   Gly Ala Leu Gln Gly Leu Leu Arg Gln Val Asn Gln Ser Ser Val Gln
   260 265 270
   Val Val Val Leu Phe Ser Ser Ala His Ala Ala Arg Thr Leu Phe Ser
   275 280 285
   Tyr Ser Ile Arg Cys Lys Leu Ser Pro Lys Val Trp Val Ala Ser Glu
   290 295 300
   Ala Trp Leu Thr Ser Asp Leu Val Met Thr Leu Pro Gly Met Pro Gly
   305 310 315 320
   Val Gly Thr Val Leu Gly Phe Leu Gln Gln Gly Ala Pro Met Pro Glu
   325 330 335
   Phe Pro Ser Tyr Val Arg Thr Arg Leu Ala Leu Ala Ala Asp Pro Ala
   340 345 350
   Phe Cys Ala Ser Leu Asp Ala Glu Gln Pro Gly Leu Glu Glu His Val
   355 360 365
   Val Gly Pro Arg Cys Pro Gln Cys Asp His Val Thr Leu Glu Asn Leu
   370 375 380
   Ser Ala Gly Leu Leu His His Gln Thr Phe Ala Ala Tyr Ala Ala Val
   385 390 395 400
   Tyr Gly Val Ala Gln Ala Leu His Asn Thr Leu Arg Cys Asn Ala Ser
   405 410 415
   Gly Cys Pro Arg Arg Glu Pro Val Arg Pro Trp Gln Leu Leu Glu Asn
   420 425 430
   Met Tyr Asn Val Ser Phe Arg Ala Arg Gly Leu Ala Leu Gln Phe Asp
   435 440 445
   Ala Ser Gly Asn Val Asn Val Asp Tyr Asp Leu Lys Leu Trp Val Trp
   450 455 460
   Gln Asp Pro Thr Pro Glu Leu Arg Thr Val Gly Thr Phe Lys Gly Arg
   465 470 475 480
   Leu Glu Leu Trp Arg Ser Gln Met Cys Trp His Thr Pro Gly Lys Gln
   485 490 495
   Gln Pro Val Ser Gln Cys Ser Arg Gln Cys Lys Glu Gly Gln Val Arg
   500 505 510
   Arg Val Lys Gly Phe His Ser Cys Cys Tyr Asn Cys Val Asp Cys Lys
   515 520 525
   Ala Gly Ser Tyr Gln Arg Asn Pro Asp Asp Leu Leu Cys Thr Gln Cys
   530 535 540
   Asp Gln Asp Gln Trp Ser Pro Asp Arg Ser Thr Arg Cys Phe Ala Arg
   545 550 555 560
   Lys Pro Met Phe Leu Ala Trp Gly Glu Pro Ala Val Leu Leu Leu Leu
   565 570 575
   Ala Leu Leu Ala Leu Ala Leu Gly Leu Ala Leu Ala Ala Leu Gly Leu
   580 585 590
   Phe Leu Trp His Ser Asp Ser Pro Leu Val Gln Ala Ser Gly Gly Pro
   595 600 605
   Arg Ala Cys Phe Gly Leu Ala Cys Leu Gly Leu Val Cys Leu Ser Val
   610 615 620
   Leu Leu Phe Pro Gly Gln Pro Gly Pro Ala Ser Cys Leu Ala Gln Gln
   625 630 635 640
   Pro Leu Phe His Leu Pro Leu Thr Gly Cys Leu Ser Thr Phe Phe Leu
   645 650 655
   Gln Ala Ala Glu Ile Phe Val Gly Ser Glu Leu Pro Pro Ser Trp Ala
   660 665 670
   Glu Lys Met Arg Gly Arg Leu Arg Gly Pro Trp Ala Trp Leu Val Val
   675 680 685
   Leu Leu Ala Met Leu Ala Glu Ala Ala Leu Cys Ala Trp Tyr Leu Val
   690 695 700
   Ala Phe Pro Pro Glu Val Val Thr Asp Trp Arg Val Leu Pro Thr Glu
   705 710 715 720
   Ala Leu Val His Cys His Val His Ser Trp Ile Ser Phe Gly Leu Val
   725 730 735
   His Ala Thr Asn Ala Met Leu Ala Phe Leu Cys Phe Leu Gly Thr Phe
   740 745 750
   Leu Val Gln Ser Arg Pro Gly Arg Tyr Asn Gly Ala Arg Gly Leu Thr
   755 760 765
   Phe Ala Met Leu Ala Tyr Phe Ile Thr Trp Ile Ser Phe Val Pro Leu
   770 775 780
   Phe Ala Asn Val His Val Ala Tyr Gln Pro Ala Val Gln Met Gly Thr
   785 790 795 800
   Ile Leu Leu Cys Ala Leu Gly Ile Leu Ala Thr Phe His Leu Pro Lys
   805 810 815
   Cys Tyr Leu Leu Leu Gln Arg Pro Glu Leu Asn Thr Pro Glu Phe Phe
   820 825 830
   Leu Glu Asp Asn Ala Arg Ala Gln Gly Ser Ser Trp Gly Gln Gly Arg
   835 840 845
   Gly Glu Ser Gly Gln Lys Gln Val Thr Pro Asp Pro Val Thr Ser Pro
   850 855 860
   Gln
   865
<210> 3
   <211> 2526
   <212> DNA
   <213> Felis catus
<400> 3
   atgtcactcc cggcggctca cctggtcggc ctgcagctct ccctctcctg ctgctgggct 60
   ctcagctgcc acagcacaga gacgtctgcc gacttcagcc tccctgggga ttacctcctc 120
   gcaggtctgt tccctctgca ctctgactgt ccgggcgtga ggcaccggcc cacggtgacc 180
   ctctgtgaca ggcccgacag cttcaacggt cacggctacc acctcttcca ggccatgcgg 240
   tttggcatcg aggagataaa caactccacg gccctcctgc cgaacgtcac cctgggatac 300
   cagctgtacg acgtgtgctc ggagtctgcc aacgtgtatg ccacactaaa cgtgctctcc 360
   ctgctgggga cacatcacgt agagatccga gcagaccctt cccactattc gcctgccgcc 420
   ctggctgtca ttgggcctga caccaccaac cacgcagcca ccactgcagc cctgctgagc 480
   cccttcctgg tgcccctgat cagctacgag gccagcagcg tgacgctcgg agtgaagcgg 540
   cattacccct cgtttctgcg caccatcccc agcgacaagc accaggtgga ggccatggtg 600
   ctgctgctgc agagcttcgg gtgggtctgg atctcggtgg tcggcagcga cggcgactac 660
   gggcagctgg gggtgcaggc gctggaggag caggccaccc agcagggcat ctgcgttgcc 720
   ttcaaggaca tcatcccctt ctctgcccgg ccgggcgacg agaggatgca gagcatcatg 780
   caccacctgg cccgagcgag gaccaccgtt gtggtcgttt tctccagcag gcagctggcc 840
   agggtgttct ttgagtcggt ggtgctggcc aacctgactg ccaaggtgtg gatcgcctca 900
   gaagactggg ccatctctag acacatcagc aatgtgcccg ggatccaggg cattggcacg 960
   gtgctgggtg tggccatcca gcagaggctt gtccctggcc tgaaggagtt tgaagaggcc 1020
   tatgtccagg cagataaggg ggcccctggg ccttgctcca ggacctccga gtgcagcagc 1080
   aaccagctct gtagagagtg tcgggctttc acggcagagc agatgcccac gctcggggca 1140
   ttctccatga gctctgctta taacgcctac cgggcagtct acgcagtggc ccatggcctc 1200
   caccagctcc tgggctgtgc ctctggagcc tgttccaggg accgagtcta cccctggcag 1260
   cttctggagc agatccgcaa ggtgaatttc ctcctacaca aggacaccgt gaggtttaat 1320
   gacaacgggg accctctcag tggctacgac ataattgcct gggactggag tggccccaag 1380
   tggaacttca gggtcattgg ctcctccatg tggcctccag ttcagctgga cataaataaa 1440
   accaaaatcc ggtggcacgg gaaggacaac caggtgccaa agtctgtgtg ctccagcgac 1500
   tgcctcgaag ggcaccagcg agtgatttcg ggtttctacc actgttgctt tgagtgtgtg 1560
   ccctgtgagg ccgggagctt cctcaacaag agcgacctcc acagctgcca gccttgtggg 1620
   aaagaaaagt gggcacccgc gggaagtgaa acctgctttc cacgcaccgt ggtgtttttg 1680
   acttggcacg agaccatctc ttgggtgctg ctggcagcta atacgttgct gctgctgctg 1740
   gtgactggga ctgctggcct gtttgcctgg cacttagaca cccctgtggt gaagtccgct 1800
   gggggccgac tgtgcttctt catgctaggc tccctggcag ggggcagctg tgggctctac 1860
   ggcttttttg gggagcccac gctgcccaca tgcttgttgc gccaaagcct ccttgccctg 1920
   ggttttgcca tcttcctgtc ctgcctgacc atccgctcct tccaactggt cttcatcttc 1980
   aagttttctg ccaaggtacc caccttctac cgtgcctggg tccaaaacca cggtcctggc 2040
   ctatttgtgg tgatcagctc aatggcccag ctgctcatct gtctaacttg gctggcggtg 2100
   tggaccccac tgcccaccag ggagtaccag cgcttccctc agctggtggt gcttgattgc 2160
   acagaggcca actcaccggg cttcatgttg gctttcgcct acaatggcct cctgtccgtc 2220
   agcgcctttg cctgcagcta cctgggcaag gacctgccag agaactacaa cgaggccaaa 2280
   tgtgtcactt ttagtctgct gctcaacttc gtgtcctgga ttgccttctt caccacggcc 2340
   agcgtctacc agggcaagta cttgcccgcg gtcaacgtgc tggcggcgct gagcagcctg 2400
   agtggcggct tcagcggtta tttcctcccc aagtgctacg tgatcctgtg ccgcccaaaa 2460
   tttaacagca cacagcactt ccaggcctcc atccaggagt acacgaggcg ctgcggctcc 2520
   acctga 2526
<210> 4
   <211> 2598
   <212> DNA
   <213> Felis catus
<400> 4
   atgcccggcc tcgctctcct gggcctcacg gctctcctgg gcctcacggc tctcttggac 60
   cacggggagg gcgcaacgtc ctgcttgtca cagcagctca ggatgcaggg ggactatgtg 120
   ctgggtgggc tcttccctct gggctctgcc gagggtacag gtcttggcga cgggctgcag 180
   cccaatgcca ccgtgtgcac caggttctcg tctctgggcc tgctctgggc gctggccgtg 240
   aagatggcgg tggaggagat caacaacggg tcggccctgc tgcccgggct gcacctgggc 300
   tatgacctct ttgacacgtg ttcagagccc atggtggcca tgaagcccag cctcgtgttc 360
   atggccaaag caggcagctg cagcattgcc gcctactgca attacacaca gtaccagccc 420
   cgcgtgctgg ccgtcatcgg gccccactcg tctgagctcg ccctcgtcac cggcaagttc 480
   ttcagcttct tccttgtgcc tcaggtcagc tacggcgcca gcaccgaccg gctgagcaac 540
   cgggagatct tcccgtcctt cttccgcacg gtgcccagcg accaggtgca ggtggcggcc 600
   atggtggagc tgctggagga gctcggctgg aactgggtgg cggcggtggg tagtgacgac 660
   gagtatggcc ggcagggcct gagcctcttc tccggcctgg ccagcgccag gggcatctgc 720
   atcgcgcatg agggcctggt gccactgccg ccaggcagcc tgcggctggg cgccctacag 780
   ggcctgctgc gccaggtgaa ccagagcagc gtgcaggtgg tggtgctgtt ctcctccgcc      840
   cacgcggccc gcaccctctt cagctacagc atccgctgca agctctcacc caaggtgtgg      900
   gtggccagcg aggcctggct gacctcagac ctggtcatga cgctgcccgg catgcctggg      960
   gtgggcaccg tgctgggctt cctgcagcag ggcgccccga tgccggagtt cccatcctac     1020
   gtgcggaccc gcctggccct ggccgctgac cctgccttct gcgcctcgct ggacgctgaa     1080
   cagccaggcc tggaggagca cgtggtgggg ccacgctgcc cccaatgtga ccacgtcacg     1140
   ctagagaacc tatctgcggg gctgctgcac caccagacct tcgctgccta cgcggctgtg     1200
   tatggcgtgg cccaagccct tcacaacaca ctgcgctgca atgcctcggg ctgccccagg     1260
   cgggagcctg tgcggccctg gcagctccta gagaacatgt acaacgtgag cttccgtgct     1320
   cgcggcctgg cactgcagtt cgacgccagc gggaacgtga acgtggatta cgacctgaaa     1380
   ctgtgggtgt ggcaggaccc gacgcccgag ctgcgcaccg taggcacctt caagggccgc     1440
   ctggagctct ggcgctctca gatgtgctgg cacacgccgg ggaagcagca gcccgtgtcc     1500
   cagtgctccc ggcagtgcaa ggaaggccag gtgcgccgcg tgaagggctt ccactcttgc     1560
   tgttacaact gcgtggactg caaggcgggc agttatcagc gcaacccaga tgacctcctc     1620
   tgcacccagt gtgaccagga ccagtggtcc ccagaccgga gcacacgctg cttcgcccgc     1680
   aagcccatgt tcctggcatg gggggagcca gctgtgctgc tactgctcgc gctgctggct     1740
   ctggcgctgg gcctggcgct ggcagccctg gggctcttcc tctggcactc ggacagcccg 1800
   ctggttcagg cctcaggtgg gccacgggcc tgctttggcc tggcttgcct gggcctggtc 1860
   tgcctcagtg tcctcctgtt ccctggccag ccaggccctg ccagctgcct ggcccagcag 1920
   ccactgttcc acctcccact cactggctgc ctgagcacgt ttttcctgca agcggccgag 1980
   atatttgtgg ggtcggagct gccaccaagc tgggctgaga agatgcgtgg ccgcctgcgg 2040
   gggccctggg cctggctggt ggtgctgctt gctatgctgg cagaagccgc attgtgtgcc 2100
   tggtacctgg tagccttccc gccagaggtg gtgacggact ggcgggtact gcccacagag 2160
   gcgctggtgc actgccacgt gcactcctgg atcagcttcg gcctggtgca tgccactaac 2220
   gccatgctgg ccttcctctg cttcctgggc actttcctgg tgcagagccg gccaggccgc 2280
   tacaatggtg cccgcggcct cacctttgcc atgctggcct acttcatcac ctggatctcc 2340
   tttgtgcccc tctttgccaa tgtgcacgtg gcctaccagc ctgccgtgca gatgggcacc 2400
   atcctcctct gtgccctggg tatcctagcc accttccacc tgcccaagtg ctacctgctg 2460
   ctgcagcggc cggagctcaa cacccctgag ttcttcctgg aagacaatgc cagagcacag 2520
   ggcagcagtt gggggcaggg gaggggagaa tcggggcaaa aacaagtgac acccgatcca 2580
   gtgacctcac cgcagtga 2598

## Description

### TECHNICAL FIELD

The present invention relates generally to the field of taste receptor stimulation. Specifically, the present invention relates to the discovery of *in vitro* methods of stimulating the Felidae T1R1/T1R3 receptor by contacting the receptor with pyrophosphate and one or more amino acids selected from Lysine, Alanine, and Proline.

### BACKGROUND

The T1R class of taste receptors normally comprises three members: T1R1, T1R2, and T1R3. The dimer T1R2/T1R3 responds to sweet stimuli, while the dimer of T1R1/T1R3 recognizes amino acids (umami sensation in humans).

The cat and its relatives within the Felidae family possess the gene for T1R2, Taslr2, but with a micro-deletion encompassing 247 bp (basepairs) within exon 3. The 247 bp deletion causes a frameshift that generates premature stop codons in exons 4 and 6. No protein product of the Felidae Taslr2 gene can be located, making it a likely "pseudogene."

Loss of a functional Felidae T1R2 receptor implies loss of a positive dimension of flavor in cats. Novel methods and compositions for specifically and efficiently stimulating the Felidae T1R1/T1R3, which thus appears to be the sole functional T1R class receptor in felidae are highly desirable.

US 2008/244761 describes the use of human T1R1/T1R3 taste receptors in assays for identifying compounds that are capable of responding to and/or of modulating umami taste stimuli.

WO 2005/005480 identifies genes encoding T1R1, T1R2 and T1R3 taste receptors in cats.

WO 2007/118876 relates to the use of phosphate salts in Maillard reactions for preparing palatable wet petfood.

### SUMMARY

Described herein are methods for determining if two or more test compounds have an augmenting effect on the T1R1/T1R3 receptor where each test compound alone elicits a small or moderate response comprising contacting the receptor with the test compounds, and measuring the response of the receptor to the test compounds, wherein the compounds have an augmenting effect on the receptor if the response of the receptor to the compounds in a mixture in which each compound is presented at a given concentration is greater than the response to either one of the compounds alone at its given respective concentration. Responses are measured in terms of the Fluorescence Ratio F₃₄₀/F₃₈₀ as detailed in the Examples. Responses are gauged by comparing each response with the response to 100 µM isoproterenol, the positive control which activates the cell through Gα15. A null response is less than 10% of the control response. A small or moderate response is equal to or greater than 10% of the control response. In particular, the T1R1/T1R3 receptor is a Felidae T1R1/T1R3 receptor

In particular embodiments of the herein-described methods for determining if two or more test compounds have an augmenting effect on the T1R1/T1R3 receptor, the response of the receptor to the test compound is measured by calcium imaging. In particular herein-described embodiments, the calcium imaging is conducted with a fluorescent dye, including but not limited to acetoxymethyl esters, for example, Calcium Green 1-AM™, Fura2-AM™, and Fura Red AM™.

In particular embodiments of the herein-described methods for determining if two or more test compounds have an augmenting effect on the T1R1/T1R3 receptor, a cell-based assay is employed to measure the response of the receptor to the test compound. In further particular herein-described embodiments, the cell-based assay comprises HEK293-Gα15 cells that express one or more polynucleotides encoding a Felidae T1R1/T1R3 receptor. In yet further particular herein-described embodiments, the cell-based assay comprises HEK293- Gα15 cells that are transfected with one or more polynucleotides encoding a Felidae T1R1/T1R3 receptor.

In particular embodiments of the herein-described methods for determining if two or more test compounds have an augmenting effect on the T1R1/T1R3 receptor, one or more test compounds is an amino acid. In some particular herein-described embodiments, one test compound is pyrophosphate. In some particular herein-described embodiments, the one or more amino acids is present at a final concentration equal to or greater than 1 mM and/or the pyrophosphate is present at a final concentration equal to or greater than 1 mM. In some particular herein-described embodiments, the one or more amino acids is present at a final concentration equal to or greater than 1 mM and/or the pyrophosphate is present at a final concentration equal to or greater than 5 mM. In some particular herein-described embodiments, the one or more amino acids is present at a final concentration equal to or greater than 1 mM and/or the pyrophosphate is present at a final concentration equal to or greater than 10 mM. In some particular herein-described embodiments, the one or more amino acids is present at a final concentration equal to or greater than 1 mM and/or the pyrophosphate is present at a final concentration equal to or greater than 20 mM. In further particular herein-described embodiments, the one or more amino acids is present at a final concentration equal to or greater than 1 mM and/or the pyrophosphate is present at a final concentration equal to or greater than 25 mM. In yet further particular herein-described embodiments, the one or more amino acids is present at a final concentration equal to or greater than 1 mM and/or the pyrophosphate is present at a final concentration equal to or greater than 30 mM. In some particular herein-described embodiments, the one or more amino acids is present at a final concentration equal to or greater than 50 mM and/or the pyrophosphate is present at a final concentration equal to or greater than 1 mM. In some particular herein-described embodiments, the one or more amino acids is present at a final concentration equal to or greater than 50 mM and/or the pyrophosphate is present at a final concentration equal to or greater than 5 mM. In some particular herein-described embodiments, the one or more amino acids is present at a final concentration equal to or greater than 50 mM and/or the pyrophosphate is present at a final concentration equal to or greater than 10 mM. In some particular herein-described embodiments, the one or more amino acids is present at a final concentration equal to or greater than 50 mM and/or the pyrophosphate is present at a final concentration equal to or greater than 20 mM. In further particular herein-described embodiments, the one or more amino acids is present at a final concentration equal to or greater than 50 mM and/or the pyrophosphate is present at a final concentration equal to or greater than 25 mM. In yet further particular herein-described embodiments, the one or more amino acids is present at a final concentration equal to or greater than 50 mM and/or the pyrophosphate is present at a final concentration equal to or greater than 30 mM. In particular herein-described embodiments, the amino acid is proline present at a final concentration of 25 mM and/or the pyrophosphate is present at a final concentration of 20 mM. In further particular herein-described embodiments, the amino acid is proline present at a final concentration of 50 mM and/or the pyrophosphate is present at a final concentration of 20 mM. In yet further particular herein-described embodiments, the amino acid is lysine present at a final concentration of 25 mM and/or the pyrophosphate is present at a final concentration of 20 mM. In further particular herein-described embodiments, the amino acid is alanine present at a final concentration of 25 mM and/or the pyrophosphate is present at a final concentration of 20 mM. In yet further particular herein-described embodiments, the amino acid is histidine present at a final concentration of 25 mM and/or the pyrophosphate is present at a final concentration of 20 mM. In particular herein-described embodiments, the pyrophosphate is sodium pyrophosphate. In further particular embodiments, the pyrophosphate is trisodium pyrophosphate, or Na₃HP₂O₇. In some herein-described embodiments, "final concentration" means the concentration of the compound in the tasting solution superfused in the recording chamber of the calcium imaging technique.

Described herein are methods for selecting a cell line that is suitable for carrying out the methods for determining if two or more test compounds have an augmenting effect on the T1R1/T1R3 receptor. The selection methods comprise contacting one or more cell lines with isoproterenol, and conducting calcium imaging to measure the response of the endogenous isoproterenol receptor in the cell line, and selecting a cell line that exhibits a response by the endogenous isoproterenol receptor.

Also described is a method for generating a cell line expressing the T1R1/T1R3 receptor comprising contacting a cell line with isoproterenol, conducting calcium imaging to measure the response of the endogenous isoproterenol receptor in said cell line, selecting a cell line that responds to isoproterenol and transfecting the selected cell line with one or more polynucleotides encoding the T1R1/T1R3 receptor.

Described herein is a method for determining the pyrophosphate-augmented activity of a test compound on the T1R1/T1R3 receptor if each test compound alone elicits a small or moderate response comprising contacting one or more cells expressing one or more polynucleotides encoding the T1R1/T1R3 receptor with a test compound in both the absence and in the presence of pyrophosphate, wherein the compound has a pyrophosphate-enhanced activity on the receptor if the response of the receptor to the compounds in a mixture in which each compound is presented at a given concentration is greater than the response to one of the compounds alone at that given concentration. In further particular herein-described embodiments, the cells are transfected with one or more polynucleotides encoding the T1R1/T1R3 receptor.

Described herein is a method for determining the pyrophosphate-suppressive activity of a test compound on the T1R1/T1R3 receptor comprising contacting one or more cells expressing the T1R1/T1R3 receptor with a test compound in both the absence and in the presence of pyrophosphate, wherein the compound has a suppressive activity if the response to the compound and pyrophosphate is less than the smaller response to either the test compound or the pyrophosphate alone.

In particular herein-described embodiments of the previous two methods, the T1R1/T1R3 receptor is a Felidae T1R1/T1R3 receptor. In yet further particular herein-described embodiments, the response of the T1R1/T1R3 receptor was measured by calcium imaging. In particular herein-described embodiments, the cells are HEK293-Gα15 cells. In further particular herein-described embodiments, the pyrophosphate is present at a final concentration equal to or greater than 1 mM. In further particular herein-described embodiments, the pyrophosphate is present at a final concentration equal to or greater than 5 mM. In further particular herein-described embodiments, the pyrophosphate is present at a final concentration equal to or greater than 10 mM. In further particular herein-described embodiments, the pyrophosphate is present at a final concentration equal to or greater than 20 mM. In yet further particular herein-described embodiments, the pyrophosphate is present at a final concentration equal to or greater than 25 mM. In particular herein-described embodiments, the pyrophosphate is sodium pyrophosphate. In further particular herein-described embodiments, the pyrophosphate is trisodium pyrophosphate, or Na₃HP₂O₇.

Described herein is a method for adjusting a food formulation for Felidae comprising contacting one or more cells transfected with T1R1/T1R3 receptor with a test compound in both the absence and in the presence of pyrophosphate, conducting calcium imaging to measure the response of the T1R1/T1R3 receptor in the cells, wherein the compounds have an augmenting effect on the receptor if the response of the receptor to the compounds in a mixture in which each compound is presented at a given concentration is greater than the response to one of the compounds alone at its initial concentration, if each test compound alone elicits a small or moderate response, adding both the test compound that has been determined to exhibit pyrophosphate augmented activity and pyrophosphate to a current food formulation to create an adjusted food formulation, measuring the preference of Felidae for the adjusted food formulation containing the test compound and pyrophosphate, compared to the preference of the Felidae for the current food formulation without the added test compound and pyrophosphate, wherein if the Felidae show a preference for the adjusted food formulation containing the test compound and pyrophosphate, compared to the current food formulation without the added test compound and pyrophosphate, the test compound and pyrophosphate is selected for addition to food formulations for the Felidae. In particular herein-described embodiments, the cells are HEK293-Gα15 cells. In particular herein-described embodiments, the pyrophosphate is present at a final concentration equal to or greater than 1 mM. In further particular herein-described embodiments, the pyrophosphate is present at a final concentration equal to or greater than 5 mM. In further particular herein-described embodiments, the pyrophosphate is present at a final concentration equal to or greater than 10 mM. In yet further particular herein-described embodiments, the pyrophosphate is present at a final concentration equal to or greater than 20 mM. In further particular herein-described embodiments, the pyrophosphate is present at a final concentration equal to or greater than 25 mM.

Described herein is a method for determining the minimal amount of a test compound and pyrophosphate that are needed to augment the taste intensity of a food formulation for Felidae comprising contacting the T1R1/T1R3 receptor with a test compound in both the absence and in the presence of pyrophosphate, measuring the response of the T1R1/T1R3 receptor to the test compound in both the absence and in the presence of pyrophosphate, wherein if an increase in the response of the T1R1/T1R3 receptor is measured in the presence of both the test compound and pyrophosphate relative to the response of the T1R1/T1R3 receptor measured in the presence of test compound or pyrophosphate alone, the test compound is determined to exhibit pyrophosphate augmented activity, adding both the test compound that has been determined to exhibit pyrophosphate augmented activity and pyrophosphate to a current food formulation to create a taste intensity augmented food formulation, measuring the preference of Felidae for the taste intensity augmented food formulation containing the test compound and pyrophosphate, compared to the preference of the Felidae for the current food formulation without the added test compound and pyrophosphate, wherein if the Felidae show a preference for the taste intensity augmented food formulation containing the test compound and pyrophosphate, compared to the current food formulation without the added test compound and pyrophosphate, the test compound and pyrophosphate is selected for addition to food formulations for the Felidae. The method is repeated with consecutively decreasing amounts of test compound and pyrophosphate, until the minimum quantity of test compound and pyrophosphate needed for the Felidae to demonstrate a preference for the adjusted food formulation containing the test compound and pyrophosphate, compared to the current food formulation without the added test compound and pyrophosphate is determined.

Also described is a method for augmenting the animal equivalent of human umami taste in domestic animal foodstuff by adding pyrophosphate to domestic animal foodstuff comprising one or more amino acids. In particular herein-described embodiments, the one or more amino acids are Lysine, Alanine, Proline or Histidine. In further particular herein-described embodiments, the domestic animal foodstuff is a catfood.

Provided herein are *in vitro* methods for stimulating the Felidae T1R1/T1R3 receptor comprising contacting the receptor with pyrophosphate and one or more amino acids selected from Lysine, Alanine, and Proline. In some embodiments, the receptor is first contacted with pyrophosphate. In some embodiments, the receptor is first contacted with one or more amino acids. The receptor is contacted with pyrophosphate present at a final concentration equal to or greater than 20 mM. In preferred embodiments, the receptor is contacted with pyrophosphate present at a final concentration equal to or greater than 25 mM. In preferred embodiments, the Felidae T1R1/T1R3 receptor comprises the cat T1R1 and the cat T1R3. The sequences of the corresponding polypeptides and cDNAs may be found in the attached sequence listing and are listed below:
cat T1R1 polypeptide amino acid sequence: SEQ ID NO:1
cat T1R3 polypeptide amino acid sequence: SEQ ID NO:2
cat T1R1 cDNA sequence: SEQ ID NO:3
cat T1R3 cDNA sequence: SEQ ID NO:4

Described herein is a Felidae food product comprising pyrophosphate and one or more amino acids. In particular herein-described embodiments the food product contains pyrophosphate present at a final concentration that provides a final salivary concentration equal to or greater than 1 mM. In some particular herein-described embodiments the food product contains pyrophosphate present at a final concentration that provides a final salivary concentration equal to or greater than 5 mM. In further particular herein-described embodiments the food product contains pyrophosphate present at a final concentration that provides a final salivary concentration equal to or greater than 10 mM. In yet further particular herein-described embodiments, the food product contains pyrophosphate present at a final concentration that provides a final salivary concentration equal to or greater than 20 mM. In yet further particular herein-described embodiments, the food product contains pyrophosphate present at a final concentration that provides a final salivary concentration equal to or greater than 25 mM. In particular herein-described embodiments, the pyrophosphate is sodium pyrophosphate. In particular herein-described embodiments, the one or more amino acids are Lysine, Alanine, Proline or Histidine. In further embodiments herein-described, non-naturally occurring analogs or mimetics of said amino acids may be employed.

Described herein is a method for augmenting taste intensity of a cat food by adding pyrophosphate and one or more amino acids in a determined pyrophosphate: amino acid ratio. In particular herein-described embodiments, the pyrophosphate: amino acid ratio is determined by the method described *supra* for determining the minimal amount of a test compound and pyrophosphate that are needed to augment the taste intensity of a food formulation for Felidae.

Also described is a cat food product obtainable by the method described *supra* for augmenting taste intensity of a cat food by adding pyrophosphate and one or more amino acids in a determined pyrophosphate: amino acid ratio.

Described is a taste intensity augmenting agent for cat food comprising pyrophosphate and one or more amino acids in a determined pyrophosphate: amino acid ratio. In particular herein-described embodiments, the pyrophosphate: amino acid ratio is determined by the method described *supra* for determining the minimal amount of a test compound and pyrophosphate that are needed to augment the taste intensity of a food formulation for Felidae.

### BRIEF DESCRIPTION OF THE DRAWINGS

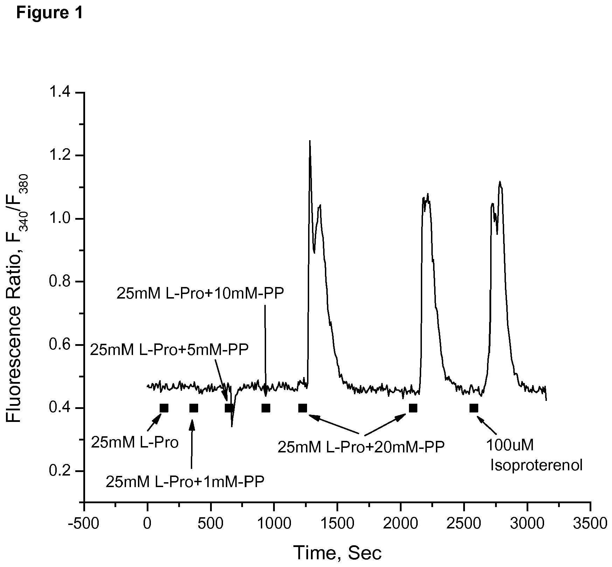

Figure 1 illustrates that an HEK293 cell expressing T1R1/T1R3 receptor and Gα15 gave null responses to 25mM L-proline (L-Pro) alone as well as mixtures of 25mM L-proline plus 1, 5, or 10mM sodium pyrophosphate (PP) but exhibited a robust response to a mixture of the two compounds (25mM L-proline plus 20mM sodium pyrophosphate). The augmented response was replicable. 100µM isoproterenol serves as positive control. The pyrophosphate employed was trisodium pyrophosphate, or Na₃HP₂O₇.

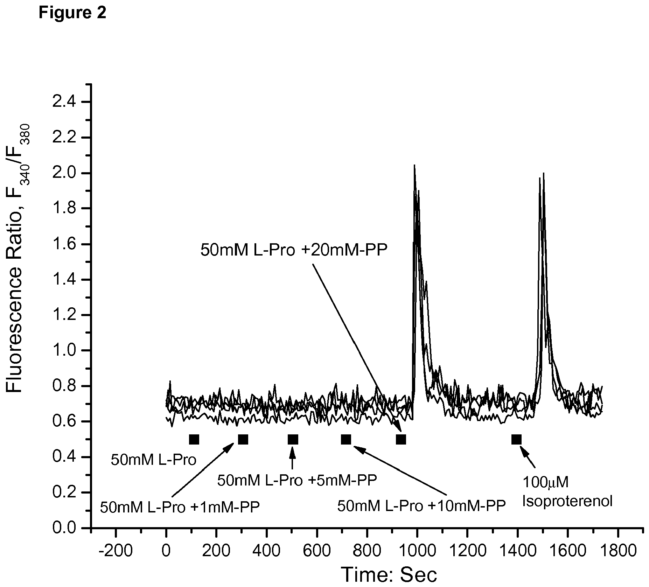

Figure 2 illustrates that 3 HEK293 cells expressing T1R1/T1R3 receptor and Gα15 gave null responses to 50mM L-proline (L-Pro) alone as well as mixtures of 50mM L-proline plus 1, 5, or 10mM sodium pyrophosphate (PP) but exhibited a robust response to a mixture of the two compounds (50mM L-proline plus 20mM pyrophosphate). 100µM isoproterenol serves as positive control.

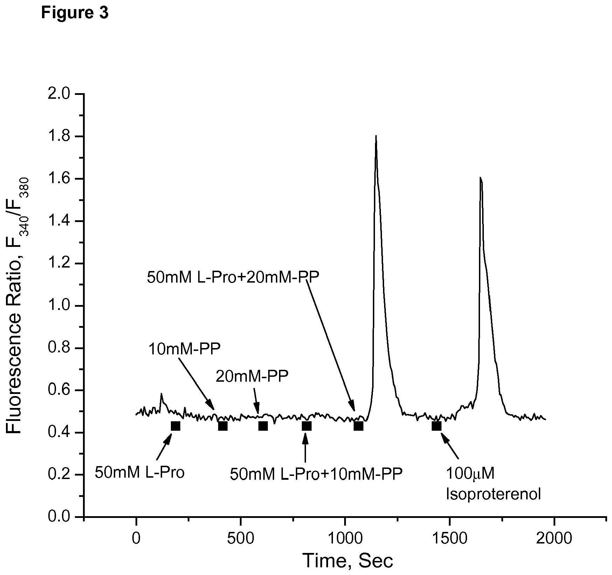

Figure 3 illustrates that an HEK293 cell expressing T1R1/T1R3 receptor and Gα15 gave null responses to 50mM L-proline (L-Pro), 10mM and 20mM sodium pyrophosphate (PP) presented singly but exhibited a robust response to a mixture of the two compounds (50mM L-proline plus 20mM sodium pyrophosphate). 100µM isoproterenol serves as positive control.

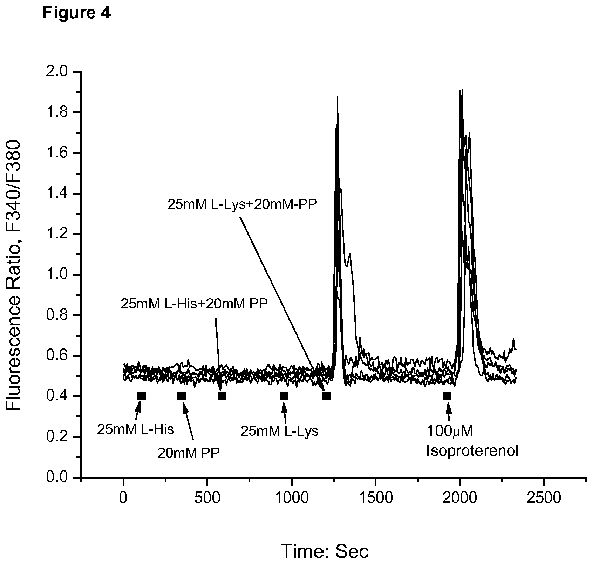

Figure 4 illustrates that 5 HEK293 cells expressing T1R1/T1R3 receptor and Gα15 gave null responses to 25mM L-histidine (L-His), 20mM sodium pyrophosphate(PP) and 25mM L-lysine (L-Lys) alone but exhibited a robust response to a mixture of 25mM L-lysine plus 20mM sodium pyrophosphate. L-histidine was not augmented in this study. 100µM isoproterenol serves as positive control.

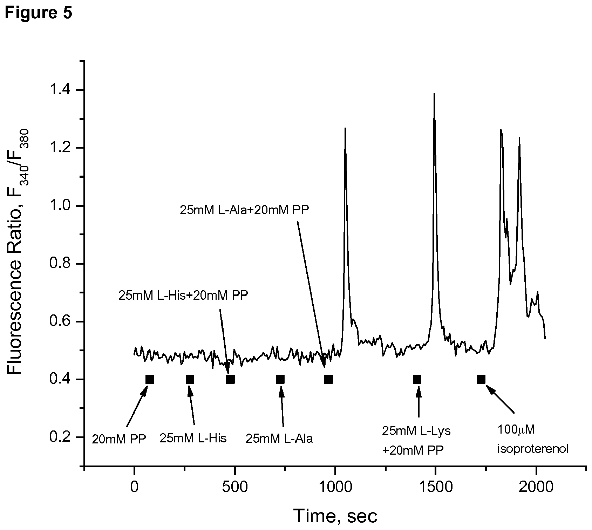

Figure 5 illustrates that a HEK293 cell expressing T1R1/T1R3 receptor and Gα15 gave null responses to 25mM L-histidine (L-His) and L-alanine (L-Ala) alone as well as mixtures of 25mM L-histidine (L-His) plus 20mM sodium pyrophosphate (PP) but exhibited a robust response to a mixture of 25mM L-alanine (L-Ala) plus 20mM sodium pyrophosphate (PP). 100µM isoproterenol serves as positive control.

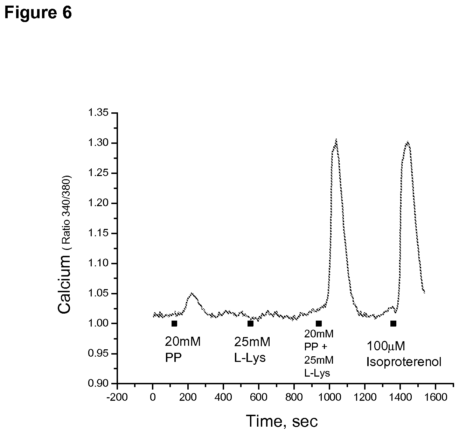

Figure 6 illustrates that a HEK293 cell expressing T1R1/T1R3 receptor and Gα15 gave a weak response to 20mM sodium pyrophosphate (PP) alone and a null response to 25mM L-lysine (L-Lys) alone but exhibited a robust response to a mixture of 20mM sodium pyrophosphate (PP) and 25mM L-lysine (L-Lys). 100µM isoproterenol serves as positive control.

### DETAILED DESCRIPTION OF ILLUSTRATIVE EMBODIMENTS

Various terms relating to aspects of the description are used throughout the specification and claims. Such terms are to be given their ordinary meaning in the art unless otherwise indicated. Other specifically defined terms are to be construed in a manner consistent with the definitions provided herein.

As used in this specification and the appended claims, the singular forms "a," "an," and "the" include plural referents unless the content clearly dictates otherwise.

The term "about" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

The term "pyrophosphate" as used herein refers to a compound with a P₂0₇ anion. In preferred embodiments, the pyrophosphate is sodium pyrophosphate. Pyrophosphates are available as di-, tri- and tetra-sodium salts (Na₂H₂P₂O₇, Na₃HP₂O₇, and Na₄P₂O₇, respectively), as well as several non-sodium forms. In the disclosed examples, the pyrophosphate employed was trisodium pyrophosphate, or Na₃HP₂O₇.

The term "Felidae" as used herein refers to a cosmopolitan family comprising lithe-bodied carnivorous mammals having often strikingly patterned fur, comparatively short limbs with soft pads on the feet, usually sharp curved retractile claws, a broad and somewhat rounded head with short but powerful jaws equipped with teeth suited to grasping, tearing, and shearing through flesh, erect ears, and typically eyes with narrow or elliptical pupils and especially adapted for seeing in dim light. Examples of Felidae comprise the domestic cat, lion, tiger, lynx, leopard, wildcat, saber-toothed tiger and cheetah.

The term "Felidae T1R1/T1R3 receptor" as used herein refers to a heterodimer of Felidae T1R1 and T1R3 polypeptides. One example of a Felidae T1R1 polypeptide comprises the amino acid sequence SEQ ID NO: 1. An example of a Felidae T1R3 polypeptide comprises the amino acid sequence SEQ ID NO: 2. The exemplary Felidae T1R1 polypeptide may be encoded by the nucleotide sequence of SEQ ID NO: 3. The exemplary Felidae T1R3 polypeptide may be encoded by the nucleotide sequence of SEQ ID NO: 4. The term "Felidae T1R1 polypeptide" refers to polymorphic variants, alleles, mutants, and interspecies homologs that: (1) have at least about 90, 95, 96, 97, 98, or 99% amino acid sequence identity to SEQ ID NO: 1; (2) specifically bind to antibodies raised against an immunogen comprising an amino acid sequence of SEQ ID NO: 1 and conservatively modified variants thereof; or (3) are encoded by a nucleic acid molecule which specifically hybridize (with a size of at least about 100, optionally at least about 500-1000 nucleotides) under stringent hybridization conditions to a sequence of SEQ ID NO: 3 and conservatively modified variants thereof. The term "Felidae T1R3 polypeptide" refers to polymorphic variants, alleles, mutants, and interspecies homologs that: (1) have at least about 90, 95, 96, 97, 98, or 99% amino acid sequence identity to SEQ ID NO: 2; (2) specifically bind to antibodies raised against an immunogen comprising an amino acid sequence of SEQ ID NO: 2 and conservatively modified variants thereof; or (3) are encoded by a nucleic acid molecule which specifically hybridize (with a size of at least about 100, optionally at least about 500-1000 nucleotides) under stringent hybridization conditions to a sequence of SEQ ID NO: 4 and conservatively modified variants thereof.

The term "amino acid" as used herein denotes a molecule containing both an amino group and a carboxyl group. In some embodiments, the amino acids are α-, β-, γ- or δ-amino acids, including their stereoisomers and racemates. As used herein the term "L-amino acid" denotes an α-amino acid having the L configuration around the α-carbon, that is, a carboxylic acid of general formula CH(COOH)(NH₂)-(side chain), having the L-configuration. The term "D-amino acid" similarly denotes a carboxylic acid of general formula CH(COOH)(NH₂)-(side chain), having the D-configuration around the α-carbon. Side chains of L-amino acids include naturally occurring and non-naturally occurring moieties. Non-naturally occurring (*i.e.,* unnatural) amino acid side chains are moieties that are used in place of naturally occurring amino acid side chains in, for example, amino acid analogs. Amino acid substituents may be attached, for example, through their carbonyl groups through the oxygen or carbonyl carbon thereof, or through their amino groups, or through functionalities residing on their side chain portions. The naturally occurring amino acids are listed in Table 1 and the unnaturally occurring amino acids are listed in Table 2 below.

**Table 1. Naturally occurring amino acids.**

| Symbol | Meaning |
|---|---|
| Ala | Alanine |
| Cys | Cysteine |
| Asp | Aspartic Acid |
| Glu | Glutamic Acid |
| Phe | Phenylalanine |
| Gly | Glycine |
| His | Histidine |
| Ile | Isoleucine |
| Lys | Lysine |
| Leu | Leucine |
| Met | Methionine |
| Asn | Asparagine |
| Pro | Proline |
| Gln | Glutamine |
| Arg | Arginine |
| Ser | Serine |
| Thr | Threonine |
| Val | Valine |
| Trp | Tryptophan |
| Tyr | Tyrosine |

**Table 2. Unnaturally occurring amino acids**

| Symbol | Meaning |
|---|---|
| Aad | 2-Aminoadipic acid |
| bAad | 3-Aminoadipic acid |
| bAla | beta-Alanine, beta-Aminopropionic acid |
| Abu | 2-Aminobutyric acid |
| 4Abu | 4-Aminobutyric acid, piperidinic acid |
| Acp | 6-Aminocaproic acid |
| Ahe | 2-Aminoheptanoic acid |
| Aib | 2-Aminoisobutyric acid |
| bAib | 3-Aminoisobutyric acid |
| Apm | 2-Aminopimelic acid |
| Dbu | 2,4-Diaminobutyric acid |
| Des | Desmosine |
| Dpm | 2,2'-Diaminopimelic acid |
| Dpr | 2,3-Diaminopropionic acid |
| EtGly | N-Ethylgycine |
| EtAsn | N-Ethylasparagine |
| Hyl | Hydroxylysine |
| aHyl | Allo-hydroxylysine |
| 3Hyp | 3-Hydroxyproline |
| 4Hyp | 4-Hydroxyproline |
| Ide | Isodesmosine |
| Alle | Allo-Isoleucine |
| MeGly | N-Methylglycine, sarcosine |
| Melle | N-Methylisoleucine |
| MeLys | 6-N-Methyllysine |
| MeVal | N-Methylvaline |
| Nva | Norvaline |
| Nle | Norleucine |
| Orn | Ornithine |

The term "null response" as used herein refers to a response that is less than 10% of the response elicited by the positive control. In preferred embodiments, the positive control is isoproterenol.

The term "small or moderate response" as used herein refers to a response that is equal to or greater than 10% of the responses elicited by the positive control. In preferred embodiments, the positive control is isoproterenol.

The term "augmenting effect" of a compound as used herein refers to the effect of a compound which when mixed at its initial concentration with another compound at its initial concentration gives a greater response than the response to either one of the compounds alone at their initial concentrations.

The term "domestic animal foodstuff" as used herein refers to commercially available solid wet or dry food for consumption by domestic companion animals

It is to be understood that the embodiments described herein are not limited to particular methods, reagents, compounds, compositions or biological systems, which can, of course, vary.

The cells employed in certain embodiments of the invention are cell lines that were transfected with or express one or more polynucleotides encoding the T1R1/T1R3 receptor and that also were transfected with or express a G protein such as Gα15 or another G protein that when expressed in association with the T1R1/T1R3 receptor produces a functional taste receptor. Cell lines that may be employed include HEK293, CHO, Jurkat, HeLa, Sf9 or other expression cell lines. The cells may be stably or transiently transfected, although stable transfection is preferred. Preferably, the cells are mammalian cells and the T1R1/T1R3 receptor is Felidae. In preferred embodiments the cells are HEK293-Gα15 cells transfected with Felidae T1R1/T1R3 receptor. The T1R1/T1R3 cDNA and protein sequences from various species, including Felidae, are provided in the sequence listing.

The responses of cells to taste stimuli are measured by using calcium imaging techniques. Calcium imaging techniques are scientific techniques that show the calcium status of a tissue, cell or medium (Nelson et al. 2001, Li et al. 2002 and Gomez et al. 2005). Transfected cells are loaded with a calcium indicator containing buffer. Preferably, the calcium indicator is a fluorescent dye, including but not limited to acetoxymethyl esters, for example Calcium Green 1-AM™, Fura2-AM™, and Fura Red AM™. In preferred embodiments, the calcium indicator is Fura2-AM™. Stimulus solutions containing the test compounds, such as amino acids and/or pyrophosphate, are applied to the cells, and calcium imaging is conducted. Preferably, coverslips with Fura2-AM™ loaded cells are placed in a recording chamber and continuously superfused with Hank's salt solution (Nelson et al. 2001) and stimulus solutions are applied by switching the superfusion. Other techniques and instruments that may be appropriate measure the calcium status of either many individual receptor-expressing cells or the calcium status of a population of receptor-expressing cells.

The methods herein-described may be used to identify test compounds that have an augmenting effect on the T1R1/T1R3 receptor, as described in the Summary of the invention. Once a test compound or a combination of test compounds has been identified that have an augmenting effect on the TlR1/T1R3 receptor, those test compounds may be selected for addition to current food formulations for Felidae, to create an adjusted food formulation. The preference of Felidae for the adjusted food formulation is measured by behavioral methods. Exemplary behavioral methods for determining preference for an adjusted food formulation include a two-bottle preference test, a lickometer, or a conditioned aversion assay. The consumption by Felidae of the adjusted food formulation is compared to the consumption by Felidae of the current food formulation without the added test compounds. If the Felidae show a preference for the adjusted food formulation containing the test compounds, compared to the current food formulation without the added test compounds, the test compounds are selected for addition to food formulations for the Felidae. In particular embodiments, the test compounds comprise one or more amino acids and pyrophosphate.

Once a test compound has been determined to exhibit pyrophosphate augmented activity by the methods described in the summary of the invention, the method may be repeated with consecutively decreasing amounts of test compound and pyrophosphate, until the minimum quantity of test compound and pyrophosphate needed for the Felidae to demonstrate a preference for the adjusted food formulation over the food formulation without the added test compound and pyrophosphate is determined.

### Examples

### Example 1

### Constructing the heterologous cell lines

### Sequencing of cat Tas1r1 and Taslr3.

The coding sequences of cat Tas1r1 and cat Taslr3 were identified by screening Felidae Genomic BAC library. The procedures for library screening and BAC sequencing were described in (Li et al. 2002; Li et al. 2005).

### Initiating heterologous expression.

**a. cDNA constructs:** To generate the full-length T1R expression constructs from cat, a PCR overlapping strategy was used to amplify coding regions of the Taslr3 gene (Horton et al. 1990). The full-length cDNA clone of Tas1r1 was obtained by screening a cat taste cDNA library. The methods for designing overgo probes and procedures for cDNA library screening were previously described (Li et al. 2005). The gene for T1R1 was cloned into expression vector pcDNA4/HisMax, and the gene for the T1R3 receptor was cloned into pCEP4. These two expression vectors have different antibiotic selections so that cells expressing receptor dimer, e.g., T1R1/T1R3, can be selected and characterized.

**b. Cell Transfection:** HEK293 cell lines that stably express Gα15 were grown and maintained at 37°C DMEM supplemented with 10% FBS and MEM nonessential amino acids (Invitrogen); the medium for Gα15 transfected cells contains 5 µg/ml blasticidin (Invitrogen). Cells were co-transfected with expression constructs by using Lipofectamine 2000 reagent (Invitrogen). T1R1/T1R3 transfected cells were selected using Zeocin (100 µg/ml) and hygromycin (400 µg/ml).

**c. Establishing stable cell lines:** Transfected cells were maintained in antibiotic selective medium until colonies appear. Single colonies were identified two weeks after transfection and were examined for RNA expression by RT-PCR and for protein expression by immunohistochemistry. Positive colonies confirmed by RT-PCR and immunohistochemistry were stocked in liquid nitrogen as stable cell lines expressing T1R1/T1R3.

### Example 2

### Method for detecting pyrophosphate enhancement.

**a.** *In* ***vitro* assays:** HEK293 cells' responses to taste stimuli were measured using calcium imaging techniques (Nelson et al. 2001, Li et al. 2002, Gomez et al. 2005). Transfected cells were loaded with fura-2 containing buffer for one hour. Coverslips with fura-2 loaded cells were then placed in a recording chamber and continuously superfused with Hank's salt solution (Nelson et al. 2001). Enhancement solutions were applied by switching the superfusion, which allowed a complete change of bath solutions within 5-20 seconds, based on preliminary experiments with fluorescent dye for each setup and recording chamber. Calcium imaging recordings were performed using Imagemaster-5 multi-wavelength quantitative fluorescence imaging system (Photon Technology International, Inc.). Cells were illuminated with UV light at two wavelengths (340 or 380 nm). Intracellular calcium activity was computed from the ratio of emitted light from the 340/380 nm wavelengths. Excitation light was shuttered between acquisitions, with exposure times being minimized (typically 50-100 msec). Images are normally acquired every 2-5 sec. Using these conditions, cells remain viable in the recording setup for more than 2 h without significant effects of dye bleaching or baseline shifts. The use of ratiometric imaging reduces the problems associated with fluorescence quenching or dye loss that can generate artifacts using single-wavelength dyes.

**b. Amino acid stimulation of the HEK transfected cell lines:** In a typical experiment to determine if there is an interaction at the receptor between two compounds, the response (peak height of intracellular calcium concentration) to each compound as well as a mixture of the two compounds is measured. If each compound alone elicits a small or moderate response, it will be necessary to compare responses to single compounds with a mixture in which each compound is presented at its initial test concentration. In this manner we can determine if the enhancement is greater than simple additivity. Suppressive interactions are simpler to detect; the response to a mixture will be less than the smaller response to each single compound. If either suppression or enhancement is detected, control experiments are carried out to rule out self-adaptation or self-sensitization.

### Example 3

### Results

The results of the HEK293 cells' responses to taste stimuli are shown in the Figures. Figure 1 illustrates that an HEK cell expressing T1R1/T1R3 receptor and Gα15 gave null responses to 25mM L-proline (L-Pro) alone as well as mixtures of 25mM L-proline plus 1, 5, or 10mM sodium pyrophosphate (PP) but exhibited a robust response to a mixture of the two compounds (25mM L-proline plus 20mM sodium pyrophosphate). The augmented response was replicable. 100µM isoproterenol serves as positive control. The pyrophosphate employed was trisodium pyrophosphate, or Na₃HP₂O₇.

Figure 2 illustrates that 3 HEK293 cells expressing T1R1/T1R3 receptor and Gα15 gave null responses to 50mM L-proline (L-Pro) alone as well as mixtures of 50mM L-proline plus 1, 5, or 10mM sodium pyrophosphate (PP) but exhibited a robust response to a mixture of the two compounds (50mM L-proline plus 20mM pyrophosphate). 100µM isoproterenol serves as positive control.

Figure 3 illustrates that an HEK293 cell expressing T1R1/T1R3 receptor and Gα15 gave null responses to 50mM L-proline (L-Pro), 10mM and 20mM sodium pyrophosphate (PP) presented singly but exhibited a robust response to a mixture of the two compounds (50mM L-proline plus 20mM sodium pyrophosphate). 100µM isoproterenol serves as positive control.

Figure 4 illustrates that 5 HEK cells expressing T1R1/T1R3 receptor and Gα15 gave null responses to 25mM L-histidine (L-His), 20mM sodium pyrophosphate(PP) and 25mM L-lysine (L-Lys) alone but exhibited a robust response to a mixture of 25mM L-lysine plus 20mM sodium pyrophosphate. L-histidine was not augmented in this study. 100µM isoproterenol serves as positive control.

Figure 5 illustrates that a HEK293 cell expressing T1R1/T1R3 receptor and Gα15 gave null responses to 25mM L-histidine (L-His) and L-alanine (L-Ala) alone as well as mixtures of 25mM L-histidine (L-His) plus 20mM sodium pyrophosphate (PP) but exhibited a robust response to a mixture of 25mM L-alanine (L-Ala) plus 20mM sodium pyrophosphate (PP). 100µM isoproterenol serves as positive control.

Figure 6 illustrates that a HEK293 cell expressing T1R1/T1R3 receptor and Gα15 gave a weak response to 20mM sodium pyrophosphate (PP) alone and a null response to 25mM L-lysine (L-Lys) alone but exhibited a robust response to a mixture of 20mM sodium pyrophosphate (PP) and 25mM L-lysine (L-Lys).

### References

Gomez G, Lischka FW, Haskins ME, Rawson NE. 2005. Evidence for multiple calcium response mechanisms in mammalian olfactory receptor neurons. Chem Senses 30: 317-26.
Horton RM, Cai ZL, Ho SN, Pease LR. 1990. Gene splicing by overlap extension: tailor-made genes using the polymerase chain reaction. Biotechniques 8: 528-35.
Li X, Bachmanov AA, Li S, Chen Z, Tordoff MG, Beauchamp GK, de Jong PJ, Wu C, Chen L, West DB, Ross DA, Ohmen JD, Reed DR. 2002. Genetic, physical, and comparative map of the subtelomeric region of mouse Chromosome 4. Mamm Genome 13: 5-19.
Li X, Li W, Wang H, Cao J, Maehashi K, Huang L, Bachmanov AA, Reed DR, Legrand-Defretin V, Beauchamp GK, Brand JG. 2005. Pseudogenization of a sweet-receptor gene accounts for cats' indifference toward sugar. PLoS Genet 1: 27-35.
McCaughey, S. A., Giza, B. K., & Tordoff, M. G. (2007). Taste and acceptance of pyrophosphates by rats and mice. American Journal of Physiology, 292, R2159-R2167
Nelson G, Hoon MA, Chandrashekar J, Zhang Y, Ryba NJ, Zuker CS. 2001. Mammalian sweet taste receptors. Cell 106: 381-90.

## Claims

1. An *in vitro* method for stimulating the Felidae T1R1/T1R3 receptor comprising:
contacting said receptor with pyrophosphate and one or more amino acids selected from Lysine, Alanine, and Proline, said pyrophosphate being present at a final concentration equal to or greater than 20mM,
thereby eliciting a response of said receptor.

2. The method of claim 1 wherein the receptor is first contacted with pyrophosphate.

3. The method of claim 1 wherein the receptor is first contacted with one or more amino acids.

4. The method of anyone of claims 1 to 3, wherein said final concentration of pyrophosphate is equal to or greater than 25 mM.

5. The method of anyone of claims 1 to 4, wherein said Felidae receptor is a cat receptor.

## Patentansprüche

1. *In vitro*-Verfahren zum Stimulieren des Felidae-T1R1/T1R3-Rezeptors, umfassend:
Kontaktieren des Rezeptors mit Pyrophosphat und einer oder mehreren Aminosäuren, die aus Lysin, Alanin und Prolin ausgewählt werden, wobei Pyrophosphat in einer Endkonzentration von größer oder gleich 20 mM vorhanden ist,
wodurch eine Reaktion des Rezeptors hervorgerufen wird.

2. Verfahren nach Anspruch 1, wobei der Rezeptor zuerst mit Pyrophosphat kontaktiert wird.

3. Verfahren nach Anspruch 1, wobei der Rezeptor zuerst mit einer oder mehreren Aminosäuren kontaktiert wird.

4. Verfahren nach einem der Ansprüche 1 bis 3, wobei die Endkonzentration von Pyrophosphat größer oder gleich 25 mM ist.

5. Verfahren nach einem der Ansprüche 1 bis 4, wobei der Felidae-Rezeptor ein Katzenrezeptor ist.

## Revendications

1. Procédé *in vitro* pour la stimulation du récepteur T1R1/T1R3 des félidés comprenant :
la mise en contact dudit récepteur avec du pyrophosphate et un ou plusieurs acides aminés choisis parmi la lysine, l'alanine, et la proline, ledit pyrophosphate étant présent à une concentration finale supérieure ou égale à 20 mM,
déclenchant de cette façon une réponse dudit récepteur.

2. Procédé selon la revendication 1, dans lequel le récepteur est d'abord mis en contact avec du pyrophosphate.

3. Procédé selon la revendication 1, dans lequel le récepteur est d'abord mis en contact avec un ou plusieurs acides aminés.

4. Procédé selon l'une quelconque des revendications 1 à 3, dans lequel ladite concentration finale du pyrophosphate est supérieure ou égale à 25 mM.

5. Procédé selon l'une quelconque des revendications 1 à 4, dans lequel ledit récepteur des félidés est un récepteur du chat.
